(19)

Europäisches Patentamt

European Patent Office

Office européen des brevets

(11) **EP 4 095 863 A1**

(12) **EUROPEAN PATENT APPLICATION**

(43) Date of publication:
**30.11.2022 Bulletin 2022/48**

(21) Application number: **22175892.3**

(22) Date of filing: **27.05.2022**

(51) International Patent Classification (IPC):
**G16B 30/00** (2019.01)     **G16B 40/20** (2019.01)

(52) Cooperative Patent Classification (CPC):
**G16B 30/00; G16B 40/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2021 GB 202107714**
**22.06.2021 GB 202108956**

(71) Applicant: **InstaDeep Ltd**
**London W2 1AY (GB)**

(72) Inventor: **Lopez Carranza, Nicolás**
**London (GB)**

(74) Representative: **EIP**
**Fairfax House**
**15 Fulwood Place**
**London WC1V 6HU (GB)**

(54) **MACHINE LEARNING FOR AMINO ACID CHAIN EVALUATION**

(57)     A computer-implemented method for evaluating an amino acid chain is provided. The method includes obtaining first data including a representation of an amino acid chain and performing a process to generate second data comprising a set of one or more probability values. The representation comprises a sequence of two or more letters, each letter representing a respective amino acid. The second process comprises, for a said position in the sequence of letters, applying a language models to the sequence of letters to determine at least one probability value associated with the said position, wherein the language model is trained using one or more datasets representing amino acid chains. A computer system configured to implement the method, and a non-transitory computer-readable storage medium, storing instructions for implementing the method, is also provided.

FIG. 1

**Description**

Technical Field

The present invention relates to evaluating amino acid chains and in particular, but not exclusively, to applying machine learning to the evaluation of amino acid chains for drug development.

Background

Proteins are large biomolecules, or macromolecules, that are made of chains of amino acids linked together by peptide bonds. In some cases, it may be desirable to change some of the amino acids in a protein in order to modify the characteristics of the protein. For example, when developing new drugs, such as a treatment for Influenza, it may be desirable to design a protein that has greater binding affinity with Influenza virus HA protein than the human receptor protein.

The underlying biological and chemical processes which govern the sequence of amino acids in a protein and their relative three-dimensional structure make evaluating and modifying proteins a labour-intensive task. Given that proteins are usually composed of a number N of amino acids ranging from 50 to 2000, there may be a large number of positions in a protein chain that could in theory be modified in an attempt to change a given characteristic of the protein.

Summary

According to a first aspect of the present invention, there is provided a computer-implemented method for evaluating an amino acid chain, the computer implemented method comprising: obtaining first data, wherein the first data includes a representation of an amino acid chain, the representation comprising a sequence of two or more letters, wherein each letter of the sequence of letters corresponds to a respective amino acid of a set of possible amino acids and a position of each letter in the sequence of letters represents a respective position of a said amino acid in the amino acid chain; and performing a process to generate second data, the second data comprising a set of one or more probability values associated with at least one position in the amino acid chain, the process comprising, for a said position in the sequence of letters, applying a language model to the sequence of letters to determine at least one probability value associated with the said position, wherein the language model is trained using one or more datasets representing amino acid chains.

Applying a language model which has been trained on data sets representing known amino acid chains to evaluate a given amino acid chain allows embedded information, relating to the underlying chemical and biological structure and characteristics of known amino acid chains, to be used to evaluate the given amino acid chain efficiently and without having to model the underlying chemical and biological principles which govern the structure of amino acid chains. By evaluating amino acid chains in this way, to determine probability values associated with given positions in the amino acid chain, it is possible to identify amino acids in the amino acid chain which are promising candidates for being modified in order to change the characteristics of the amino acid chain according to one or more criteria. For example, where a probability value associated with a given amino acid for a respective position in an amino acid chain is relatively low, this may indicate that the given amino acid is unlikely to occur at this position and so this amino acid in the chain is a promising candidate for being modified. Additionally, these probability values may also indicate whether the amino acid chain being evaluated has a high likelihood of occurring, either by synthesizing the amino acid chain or being naturally occurring. This can be used to determine whether the amino acid chain is a viable candidate for drug development.

In some embodiments, performing the process for the said position comprises masking a said letter at the said position, and applying the language model to the sequence of letters includes applying the language model to the sequence of letters with the said letter masked. In this way, the language model may be applied to the sequence of letters to determine probability values associated with possible amino acids, without knowing which of the amino acids is present in the amino acid chain at the said position.

In some embodiments, performing the process to generate second data comprises performing the process for each position in the sequence of letters to determine one or more probability values for each position. This allows each individual amino acid in the amino acid chain to be evaluated.

In some embodiments, performing the process to generate second data comprises performing the process for each position in the sequence of letters to determine two or more probability values for each position, wherein each probability value associated with a said position is associated with a different one of the set of possible amino acids from other probability values associated with the said position. This allows the embedded information derived from the amino acid chain datasets to be used in determining the viability of potential alternative amnio for one or more positions in the amino acid chain. While other methods may be employed to determine alternative amino acid candidates for each position, applying a language model in this way allows an estimation of promising amino acid candidates to be determined quickly and efficiently, with respect to computing power.

In some embodiments, performing the process to generate second data comprises performing the process for each position in the sequence of letters to determine one or more probability value for each position, wherein the one or more probability value for each position are probability values of a first type and include probability values associated with a respective letter in the sequence of letters for each position, and wherein the method comprises determining a probability value of a second type based on the probability values associated with the respective letter for each position.

Probability values of the first type represent conditional probabilities that a given amino acid is found at a respective position in the amino acid chain given that the rest of the amino acid chain comprises the sequence of amino acids as expressed in the representation of the first data. The rest of the amino acids in the amino acid chain considered in the first type of probability values may include amino acids preceding the respective position and proceeding the respective position. The probability value of the second type may provide an estimation of a sequence probability, which is a likelihood that the amino acid chain is synthesizable or would be able to occur naturally. Determining a probability value of the second type in this way allows users of the method to test the viability of given amino acid chains, such as proteins, during research phases of drug development without having to attempt to physically synthesize the amino acid chain. Applying the method in this way allows the system to provide an estimation of the viability of certain amino acid chains, thereby shortening the research phase of drug development by ruling out amino acid chains which are very unlikely, for example, which may be physiologically implausible.

In some embodiments, the probability value of the second type is determined based on a product of the probability values associated with the respective letter for each position. Taking a product of the probability values associated with the respective letters for each position in the sequence of letters provides probability values which accurately reflect the likelihood, e.g. the physiological plausibility, of the overall amino acid chain to exist.

In some embodiments, the probability of the second type is determined based on a sum of log functions of each of the probability values associated with the respective letter for each position. Taking a log function of the probability values and summing the results provides data representing the overall likelihood of the amino acid chain which is more easily processed and compared between different amino acid chains.

In some embodiments, the probability value of the second type is a first probability value of the second type, and the method further comprises: generating a second probability value of the second type associated with an amino acid chain which is different to the amino acid chain represented in the first data; and generating third data representing a comparison of the first probability value of the second type and the second probability value of the second type. Generating third data in this way allows a user of the method to efficiently compare the likelihoods of the two or more amino acid chains. In some cases, the third data may represent a comparison of more than two amino acid chains. For example, one hundred amino acid chains may all be processed according to the method and a probability value of the second type may be determined for each of the amino acid chains. The third data may include an ordered list of the amino acid chains according to their respective probability values of the second type. In other words, the third data may include an ordered list of amino acid chains which are ordered according to an estimation of their physiologically plausibility. This allows a researcher to target their research on amino acid chains which are more likely to be synthesizable and/or naturally occurring.

In some embodiments, the method comprises selecting one or more positions, and wherein the step of performing the process to generate second data comprises performing the process for the selected one or more positions to determine one or more probability value for each selected position. Applying the language model to select positions in the amino acid chain allows the application of the language model to be targeted to amino acids, or positions, of interest in the amino acid chain. This selection may utilise the output of one or more alternative evaluation algorithms thereby increasing the efficiency of evaluating the amino acid chains and allowing information regarding the amino acid chain to be determined more quickly and using less computing power.

In some embodiments, performing the process to generate the second data comprises performing the process for the selected one or more positions to determine two or more probability values for each position, wherein each probability value associated with a said position is associated with a different one of the set of possible amino acids from the other probability values associated with the said position. This allows a comparison of potential amino acids for each of the selected positions to be determined when evaluating the amino acid chain.

In some embodiments, the method further comprises generating fourth data comprising a representation of one or more alternative amino acid chains from the first data using the second data. Generating an estimation of alternative amino acid chains, e.g. having a relatively high physiological plausibility, in this way can provide candidate amino acid chains which are to be the target of research to be efficiently generated. While the alternative amino acid chains may not themselves be ideal amino acid chains, providing the characteristics which a researcher is interested in, they provide promising candidates for research which can be processed using one or more optimization algorithms to generate amino acid chains which are candidates for drug development.

In some embodiments, generating the fourth data comprises determining one or more alternative amino acid chains by: determining a first ordered list of amino acids associated with a first selected position, the first ordered list being ordered according to probability values associated with each of the amino acids for the first selected position; determining a second ordered list of amino acids associated with a second selected position, the second ordered list being ordered according to probability values associated with each of the amino acids for the selected position; and generating one or more alternative amino acid chains by selecting amino acids from the first ordered list and the second ordered list, wherein the selection prioritises amino acids for each position according to the associated probability values.

Performing a determination of alternative amino acid chains in this way allows a set of alternative amino acid sequences to be generated without having to perform a compute intensive assessment or optimization of the amino acid chains. An estimation of likely candidates for alternative amino acid chains determined in this was provides hints and pointers to alternative amino acid chains which may be used in drugs.

In some embodiments, the applying the language model comprises selecting the language model from a set of one or more language models. In some examples, a plurality of language models may be usable when evaluating amino acid chains according to the method described above, for example, ProtBert, ProtBert-BFD, and ESM-1b. These different language models may be trained on different datasets and tuned for different applications, hence the appropriateness of each of these models may differ depending on the amino acid chain represented in the first data. For example, ProtBert may be more relevant to human type amino acid chains than ProtBert-BFD. By selecting between different language models in the method, it is possible to tune the method to each use case, thereby increasing the accuracy and applicability of the results.

In some embodiments, the language model is selected based on the first data. For example, the first data may indicate the type of amino acid to which the method is to be applied and hence can be used to select a most appropriate language model to use. The first data may indicate one or more criteria of an amino acid chain represented therein, which can be used to select the language model. These criteria may represent distinguishing characteristics of the amino acid chain, such as type, species, length, utility, and so forth.

In some embodiments, the language model comprises a Transformer model including at least an encoder and trained using the one or more datasets representing amino acid chains. Transformer models are adept at language processing and allow a full sequence of letters to be evaluated in a way which is conscious of the order and arrangement of the specific letters included in a sequence of letters representing an amino acid chain. Transformers are also able to be efficiently parallelized across processors thereby allowing the method to be performed faster than when applying alternative language models.

In some embodiments, the Transformer model is trained by: providing the Transformer model with a set of masked amino acid chains, each masked amino acid chain comprising a known amino acid chain in which at least one amino acid is masked; and training the Transformer model to identify a respective set of known amino acid chains.

In some embodiments, an output of the Transformer model is input to a softmax function, and the softmax function is dependent on a temperature value. By providing a softmax function which is dependent on a temperature value it is possible to tune the distribution of probability values generated for a given position in the amino acid chain. Where the probability values are distributed evenly and it is difficult to determine whether any of the possible amino acids dominate for the given position, reducing the temperature value can reduce the noise and thereby modify the probability values to show if one probability value dominates compared to the others. Alternatively, where one probability value dominates it may be difficult to determine any information regarding the other probability values. In this case, increasing the temperature value can increase the noise and thereby reduce the dominating probability value.

In some embodiments, the method comprises obtaining a selection of a temperature value for use in the softmax function. Allowing a selection of the temperature value provides a method for tuning the method 100 according to the specific implementation of the method 100 to an amino acid chain.

According to a second aspect of the present invention, there is provided a computer system comprising at least one processor and at least one storage, the storage including: a trained language model which has been trained using one or more datasets representing amino acid chains; and computer-executable instructions which, when executed by the at least one processor, cause the computer system to perform a computer-implemented method according to the first aspect.

In some embodiments, the computer system includes one or more user interfaces. A user interface allows the interaction of a user with the computer system to provide input such as providing first data, selecting a temperature value, selecting a language model, and so forth.

According to a third aspect of the present invention, there is provided a non-transitory computer-readable storage medium comprising computer-executable instructions which, when executed by one or more processors, cause the processors to perform a computer-implemented method according to the first aspect.

Further features and advantages of the invention will become apparent from the following description of preferred embodiments of the invention, given by way of example only, which is made with reference to the accompanying drawings.

Brief Description of the Drawings

Figure 1 is a flow chart of a method for evaluating an amino acid chain according to an example;
Figure 2 is a diagram of a process included in the method according to an example;
Figure 3 is a diagram illustrating part of the method according to an example including determining a second type of probability value;
Figure 4 is a flow chart illustrating a method according to an example in which third data is generated;
Figure 5 is a diagram illustrating an example of the method in which the second data comprises a plurality of probability values for each position;
Figure 6 is a flow diagram illustrating an example of the method in which one or more alternative amino acid chains are determined;
Figure 7 shows a first ordered list of amino acids for a first position and a second ordered list of amino acids for a second position according to examples;
Figure 8 is a diagram showing a directed graph used in determining one or more alternative amino acid chains according to examples;
Figure 9 is a diagram showing an architecture of a language model according to an example;
Figure 10 is a schematic diagram of a computer system according to an example; and
Figure 11 is a schematic diagram of a non-transitory computer-readable storage medium according to an example.

Detailed Description

Details of systems and methods according to examples will become apparent from the following description, with reference to the Figures. In this description, for the purpose of explanation, numerous specific details of certain examples are set forth. Reference in the specification to "an example" or similar language means that a particular feature, structure, or characteristic described in connection with the example is included in at least that one example, but not necessarily other examples. It should further be noted that certain examples are described schematically with certain features omitted and/or necessarily simplified for ease of explanation and understanding of the concepts underlying the examples.

When developing new proteins, for example, in order to create new drugs, a known protein may be selected as a starting point. Modifications are made to the known protein in order to change the characteristics of the protein according to one or more criteria. Proteins generally have an amino acid chain length of between 50 and 2000, where any one of 20 possible amino acids may be present at each of the 50 to 2000 positions in the amino acid chain. Given the large number of positions in an amino acid chain and the number of possible amino acids, the total number of variations of a protein which could be produced are often too high to reasonably compute and test individually. Methods described herein aim at evaluating an amino acid chain to provide information which can be used to identify one or more amino acids in the chain which are promising starting points for modification when attempting to develop a new amino acid chain, such as a protein, according to one or more criteria. The methods described herein attempt to leverage machine learning techniques which can apply embedded information from datasets representing amino acid chains to evaluate any given amino acid chain. Such methods are able to provide information which is representative of underlying characteristics of an amino acid chain, and which can be utilised when developing new amino acid chains. In some cases, these methods may also be used to generate alternative amino acid chains based on this information. In certain examples, the methods described herein include determining probability values which provide estimations of the physiologically plausibility of given amino acid chains.

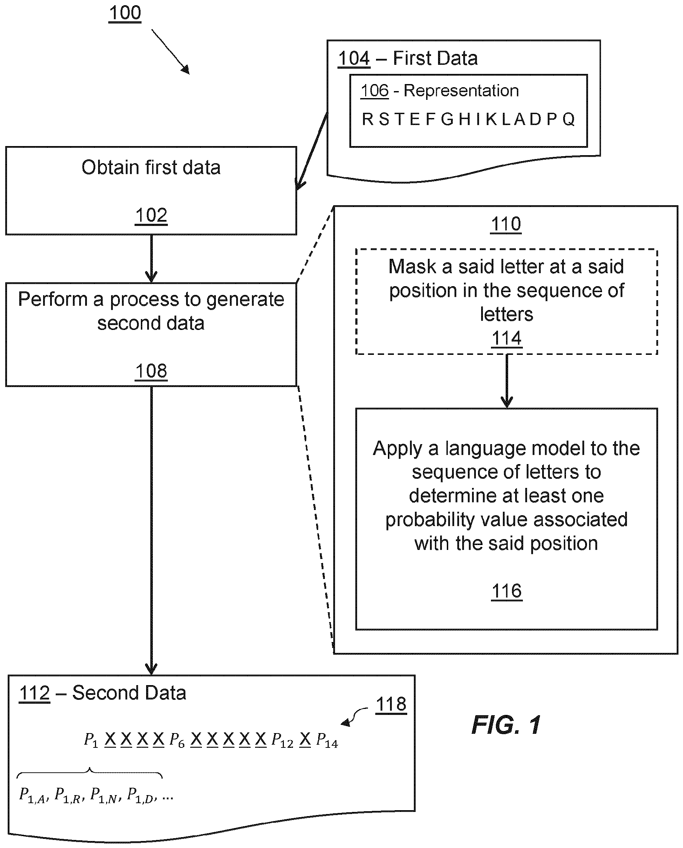

An example of a computer-implemented method for evaluating an amino acid chain according to the present disclosure is illustrated in the flow diagram of Figure 1. The method 100 includes obtaining 102 first data 104, which includes a representation 106 of an amino acid chain, and performing 108 a process 110 to generate second data 112 comprising a set of one or more probability values 118 associated with at least one position in the amino acid chain.

The representation 106 of the amino acid chain comprises a sequence of two or more letters. Each letter of the sequence of letters corresponds to a respective amino acid of a set of possible amino acids and a position of each letter in the sequence of letters represents a respective position of the respective amino acid in the chain. Table 1 shows a set of possible amino acids which may be present at each position in the amino acid chain.

*Table 1. Amino Acids and associated letter*

| AMINO ACID | ASSOCIATED LETTER |
|---|---|
| Alanine | A |

(continued)

| AMINO ACID | ASSOCIATED LETTER |
|---|---|
| Arginine | R |
| Asparagine | N |
| Aspartic acid | D |
| Cysteine | C |
| Glutamine | Q |
| Glutamic acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | w |
| Tyrosine | Y |
| Valine | V |

The first data 104 may be obtained in the form of a Protein Data Bank (pdb) file. The pdb file format is a textual file format used to describe a three-dimensional structure of molecules. Pdb files may be obtained from the set of pdb files maintained in the Protein Data Bank, the Protein Data Bank being a database maintained by the Worldwide Protein Data Bank™. Alternatively, a pdb file may be generated from other places, or created by a user of the system, and provided as the first data 104. In other examples, the first data 104 may be a plain text file including the sequence of letters. In cases where the first data 104 is provided in another type of file format, for example as a plain text file including the sequence of letters, the first data 104 may be derived from a PDB file. It is to be appreciated that the first data 104 may be provided in other file formats which are suitable for the methods described herein, as will become apparent to those skilled in the art.

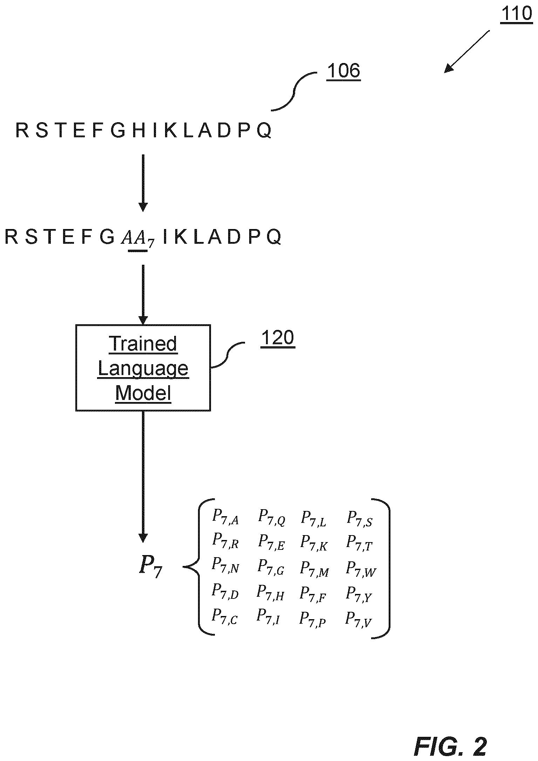

Referring also to Figure 2, the process 110 performed to generate second data 112 includes, for a given position $AA_7$ in the sequence of letters, applying 116 a language model 120 to determine at least one probability value $P_7$ associated with the position $AA_7$. The language model 120 is trained using one or more datasets representing amino acid chains such that it can recognise patterns of amino acids which commonly occur in the amino acid chains included in the datasets. For example, there may be certain patterns of amino acids which commonly occur across proteins of a certain type or all proteins.

Training the language model 120 to identify common patterns of amino acids allows the language model to infer whether certain patterns of amino acids are to be expected in other amino acid chains, outside of the datasets used for training. The occurrence of certain patterns of amino acids in an amino acid chain, such as a protein, may be governed by underlying chemical and biological processes which underpin the structure and/or function of the protein. These patterns of amino acids may frequently occur across the amino acid chains, or proteins, in the training datasets as they give some function to these proteins, such as providing stability, increasing binding to certain parts of further proteins, and so forth.

Identifying common patterns of amino acids enables the language model 120, when applied to a new amino acid chain, to determine whether these common patterns of amino acids are wholly or partially found in the new amino acid chain. The language model 120 can also infer from this determination whether there are amino acids within the new amino acid chain which disrupt, or do not conform to, these common patterns. That is to say, the language model 120 leverages the information about patterns of amino acids it has learned from the training datasets to determine the likelihood of a given amino acid, such as an amino acid represented by "H", being found at a particular position $AA_7$ in a new amino acid chain. The language model 120 does this by evaluating the amino acids found at other positions in the amino acid chain and inferring the likelihood of the given amino acid "H" being found at the position $AA_7$. For example, where an amino acid is found in the middle of a common pattern of amino acids, but does not conform to this pattern, the language model 120 may determine that this amino acid is poorly placed and/or that there is a more suitable amino acid for this position.

The language model 120 may also be able to identify whether these common patterns of amino acids are more likely to occur at certain parts of amino acid chains than at other parts of the amino acid chains. For example, some patterns of amino acids may usually be found at the end, or beginning, of an amino acid chain, and rarely found at the middle of an amino acid chain. In some cases, certain patterns of amino acids may be more likely to occur before or after certain other patterns of amino acids.

Rather than provide a binary determination of whether a given amino acid, such as "H", should be found at a given position $AA_7$, the language model 120 is able to determine a likelihood, expressed as a probability value $P_{7,H}$, that the amino acid "H" is expected to occur at this position $AA_7$. The language model 120 determines the at least one probability value $P_7$ for the position $AA_7$ by evaluating the sequence of letters representing the amino acids in the amino acid chain and inferring the suitability of a given amino acid "H" for the position $AA_7$. The language model 120 does this by leveraging the information it has learned about common patterns of amino acids from the training datasets.

The language model 120 may be configured to determine these probability values 118 based on a linguistic evaluation of the sequence of letters using inferred knowledge from known amino acid chains used to train the model 120 without considering structural or chemical characteristics as inputs to the evaluation. That is to say, the language model 120 may evaluate the sequence of letters without considering the three-dimensional structure, orientation, or arrangement of the amino acid chain which the sequence of letters represents and/or without considering binding energies or other chemical properties arising from the arrangement of the amino acids in the chain. Evaluating the sequence of letter in this way simplifies the calculations performed and may greatly increase the efficiency of calculating the probability values 118 as compared to methods which consider the properties of the amino acid chain beyond the sequence of letters used to represent the amino acid chain.

The at least one probability value $P_7$ associated with the position $AA_7$ is representative of a likelihood that a given amino acid, represented by a particular letter, will be found at position $AA_7$ given the sequence of letters representing the rest of the amino acid chain. The at least one probability value $P_7$ associated with the position $AA_7$ is included in the set of probability values 118 in the second data 112. As described above with respect to Figure 1, the amino acid chain is represented 106 by a sequence of letters. The letters in the sequence of letters represent 106 so-called wild-type amino acids which are found in the amino acid chain. The at least one probability value $P_7$ associated with the position $AA_7$ may include a probability value $P_{7,H}$ associated with the wild-type amino acid, in this case "H", for the position $AA_7$. The at least one probability value $P_7$ associated with the position $AA_7$ may additionally, or alternatively, include probability values associated with alternative amino acids for this position $AA_7$, such as amino acids represented by "G", or "F", and so forth.

According to the example shown in Figure 2, performing the process for the given position $AA_7$ includes masking 114 a letter at the position $AA_7$ and applying the language model 120 to the sequence of letters with the letter at the given position $AA_7$ masked. In the example shown in Figure 2 the letter which is masked is the letter "H" representing the wild-type amino acid at position $AA_7$ in the representation 106 of the amino acid chain. Masking a letter at a given position may include replacing the letter with a marker, or token, which indicates that the letter has been masked.

In other examples, not illustrated, the at least one probability value $P_7$ may be determined by applying the language model 120 to the sequence of letters without masking the respective letter at position $AA_7$. That is to say that the language model 120 may be applied to the sequence of letters to determine at least one probability value $P_7$ associated with the position $AA_7$ with the letter representing the wild-type amino acid for the position $AA_7$, in this case "H", included in the sequence of letters.

Applying the language model 120 to the sequence of letters without masking the letter at the position $AA_7$ allows the calculation of the at least one probability value $P_7$ to be sensitive to the wild-type amino acid found at this position $AA_7$ in the amino acid chain which is being evaluated. The wild-type amino acid for the given position $AA_7$ may influence how the language model 120 identifies common patterns which overlap with the given position $AA_7$ and other positions in the amino acid chain. With the letter representing the wild-type amino acid included in the sequence of letters, the language model 120 may infer certain patterns in the amino acid chain which it may not otherwise be able to infer. For example, when determining a probability value $P_{7,G}$, representing a likelihood that the letter "G" could alternatively be found at position $AA_7$, knowing that the letter "H" represents the wild-type amino acid for this position $AA_7$ may influence the likelihood that the letter "G" could alternatively be found at this position $AA_7$. This is because the letter "H" may form part of a common pattern of amino acids in the amino acid chain, which the language model 120 has learned to recognise from the training datasets. In contrast to this, masking the letter "H" in the sequence of letters may impede the ability of the language model 120 when inferring certain patterns in the amino acid chain.

In this example, the at least one probability value, $P_7$, includes twenty probability values, comprising a probability value associated with the wild-type amino acid and probability values associated with alternative amino acids which could be included in the amino acid chain at position $AA_7$. While the example shown includes twenty probability values for the position $AA_7$, in some cases only one probability value may be determined, for example probability value $P_{7,H}$ which is a probability value associated with the letter "H" which is present in the sequence of letters at the seventh position. The set of one or more probability values 118 each represent a probability that a given amino acid is to be found at a respective position in the amino acid chain given the structure of the rest of the amino acid chain. These probability values may be referred to as conditional probabilities associated with respective amino acids at a given position. When applied to a specific example such as probability value $P_{7,H}$ shown in Figure 2 where the amino acid at position $AA_7$ is masked, this may be expressed as:

$$P_{7,H} = P(AA_7 = H \mid AA_1 = R, AA_2 = S, AA_3 = T, AA_4 = E, AA_5 = F,$$
$$AA_6 = G, AA_8 = I, AA_9 = K, AA_{10} = L, AA_{11} = A, AA_{12}$$
$$= D, AA_{13} = P, AA_{14} = Q)$$

$$(1)$$

In examples where the amino acid for a respective position $AA_7$ is not masked, the probability value $P_{7,H}$ may alternatively be expressed as:

$$P_{7,H} = P(AA_7 = H \mid AA_1 = R, AA_2 = S, AA_3 = T, AA_4 = E, AA_5 = F,$$
$$AA_6 = G, AA_7 = H, \ AA_8 = I, AA_9 = K, AA_{10} = L, AA_{11}$$
$$= A, AA_{12} = D, \ AA_{13} = P, AA_{14} = Q)$$

$$(2)$$

An example of a dataset which the language model 120 may be trained on is the UniRef100 dataset, also referred to as the UniProt Reference Clusters, which contains data relating to two hundred and sixteen million proteins. The UniRef100 dataset provides clustered sets of sequences from the UniProt Knowledgebase which is a central hub for the collection of functional information on proteins, aimed at providing accurate, consistent, and rich annotation. Other examples of datasets which may be used include the BFD-100 dataset which includes eight times more proteins than the UniRef100 dataset. Some datasets may be more suitable for certain applications than others. When developing drugs to treat pathogens which affect humans, language models 120 trained on particular datasets which include data representing proteins associated with the human body are likely to be more suitable than language models 120 which are trained on datasets comprising information relating to proteins associated with different species or organisms.

Applying 116 the language model 120 to the sequence of letters may include selecting the language model 120 from a set of one or a plurality of language models. Where the first data 104 comprises a representation 106 of an amino acid chain such as a protein, the first data 104 may also include metadata associated with that amino acid chain which may provide an insight into which language model should be selected. In this case selecting the language model 120 may, for instance, be based on this metadata. For example, where an amino acid represented in the first data 104 is a human protein, the language model selected may be ProtBert which is ProtTrans (a pre-trained language model) trained using the UniRef100 dataset.

The first data 104 may indicate one or more criteria associated with the amino acid chain in the representation 106. These criteria may specify, for example, a species to which the amino acid chain is relevant, a type of pathogen associated with the amino acid chain, and/or a target use for the amino acid chain. The method 100 may include selecting the language model 120 based on these one or more criteria. For example, by comparing the one or more criteria to one or more characteristics associated with each of the set of language models. The characteristics associated with each language model may include the one or more datasets on which the language model is trained, an architecture, and/or type of language model. Alternatively, the language model 120 may be selected based on an input from a user interfacing with a computer-system which is implementing the method 100.

By analysing amino acid chains using language models trained from data sets comprising representations of amino acid chains, it is possible to use embedded knowledge to identify amino acids in the amino acid chain being analysed which might be good candidates for modification. In an example, a researcher may develop a new protein comprising a plurality of amino acids which has been designed to bond to a target further protein. By evaluating the protein developed by the researcher using the method 100 it may be possible to determine whether the protein is physiologically viable. For example, by applying a language model 120 which is trained on real life proteins which are stable and naturally occurring, it may be possible to identify whether any of the amino acids in the protein developed by the researchers may cause the protein to be unstable and/or should be modified or re-evaluated.

Another use case may involve evaluating a protein such as the human ACE-2 protein to determine which of the amino acids in the protein can be modified to develop a drug which has a greater binding affinity with the Sars-Cov-2 protein. In this case, the method 100 may provide one or more probability values which can indicate which of the amino acids in the human ACE-2 protein are promising candidates for optimization whilst still allowing the synthesized protein to be physiologically viable.

While the example shown in Figure 1 shows that the set of probability values 118 making up the second data 112 includes a plurality of probability values, it will be appreciated that only one probability value may be included in the set of probability values 118. The method 100 may be applied to a single position of the representation 106 and used to determine a single probability value for that position. This may be relevant where a user of the method is attempting to evaluate the suitability of a particular wild-type amino acid at a position in the chain, or to evaluate the suitability of a particular alternative amino acid for the position in the chain.

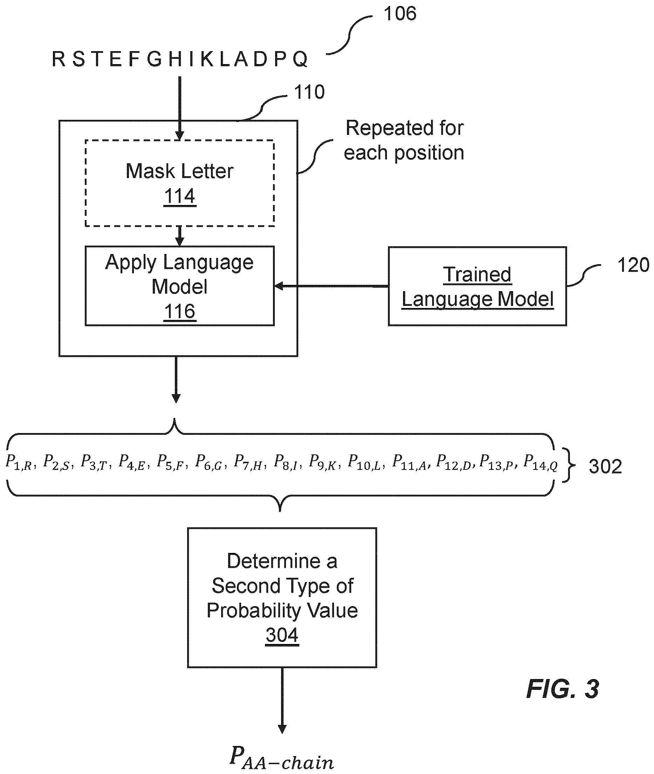

In some cases, performing 108 the process 110 to generate the second data 112 comprises performing the process 110 for each position in the sequence of letters to determine one or more probability values for each position. For example, performing the process 110 to generate second data 112 may include determining a probability value for each position, each value being associated with a respective letter in the sequence of letters, representing a respective amino acid, which is currently present at the position in the amino acid chain. This enables the evaluation of a given amino acid chain, and the viability of each of the amino acids therein, without using processing power on the consideration of alternative amino acids for each position. Figure 3 shows an example in which the process 110 is performed for each position in the sequence of letters to determine a set of probability values 302 which includes one probability value per position. In Figure 3, each probability value in the set 302 is associated with a respective position and with a respective letter representing an amino acid, which is currently present in the sequence of letters. The probability values shown in Figure 3 include a subscript which specifies the position and letter, representing an amino acid, to which the probability value is associated. For example, $P_{1, R}$ is a probability value associated with the first position in the sequence of letters and with the letter R, which represents Arginine.

The probability values derived for each position, and included in the set 302, can be used to determine an estimation that the amino acid chain represented by the sequence of letters is likely to be able to exist in real life, either by natural processes or synthesized in a laboratory. In some examples, the probability values 302 are probability values of a first type, representing a likelihood that a given amino acid would be found at a respective position in the amino acid chain given the structure of the rest of the amino acid chain as expressed in equation (1) or equation (2). The first type of probability values are conditional probabilities, representing a likelihood that a given amino acid will occur at a respective position in the amino acid chain on the condition that the amino acid chain contains the rest of the amino acids in the representation 106. The rest of the amino acids in the amino acid chain may include amino acids which precede the respective position in the amino acid chain and amino acids which proceed the respective position in the amino acid chain. The method 100 may also include determining 304 a probability value, $P_{AA\text{-}chain}$, of a second type based on probability values of the first type. In particular, probability values of the first type which are associated with the letter representing the wild-type amino acid for each position in the sequence of letters, as included in the set of probability values 302. The probability value, $P_{AA\text{-}chain}$, of the second type is an estimation of a Sequence Probability, which represents a likelihood that the amino acid chain which is being evaluated is naturally occurring or viable for synthesis.

For example, probability value $P_{1,R}$, which is of the first type, represents a likelihood that letter R, representing Arginine, would be found at the first position in an amino acid chain given the contents of the rest of the amino acid chain which is able to be represented by the sequence of letters S T E F G HI K LAD P Q. The second type of probability value, which is determined using the set of probability values 302, may represent an overall likelihood of the existence of the amino acid chain, represented 106 by the sequence of letters. It becomes possible to evaluate the viability of a given amino acid chain by leveraging data representing known amino acid chains and proteins because datasets representing known proteins and amino acid chains can provide an indication of which sequences of amino acids are likely to exist in nature. If an amino acid chain being evaluated includes sizeable sections of the chain where the particular sequence of amino acids found in this chain are rarely or never found in proteins and amino acids included in the datasets, then they are unlikely to occur naturally, and/or may be difficult to synthesize. In particular, embedded information relating to the underlying biological and chemical processes, which dictate the structure of amino acid chains, can be leveraged when evaluating a new amino acid chain without having to directly evaluate the biological and chemical processes governing the structure of the new amino acid chain. Determining a second type of probability value $P_{AA\text{-}chain}$, which represents an estimation of a Sequence Probability, or overall likelihood of the viability of the amino acid chain, also provides a criterion which can be used as an optimization criterion when developing new proteins. Applying a language model to determine information about amino acid chains in this way is a time and computing power efficient way of deriving such information during research phases of drug development.

When evaluating the likelihood of a certain amino acid chain being stable, a Sequence Probability, representing a likelihood of an amino acid chain being physiologically viable, either by naturally occurring and/or synthesis, may be determined. An expression of the Sequence Probability is shown in the equation (3) below:

$$Sequence\ Probability\ (SP) = P(AA_1 = aa_1, AA_2 = aa_2, AA_3 = aa_3, ....|)$$

$$(3)$$

where $AA_x$ represents the position of an amino acid in the amino acid chain, and $aa_x$ represents the wild-type amino acid found at that respective position in the amino acid chain. An example of the Sequence Probability is applied to a simple chain of amino acids, which can be represented by the sequence of letters [ M A A E P], in the equation (4) below:

$$SP = P(AA_1 = M, AA_2 = A, AA_3 = A, AA_4 = E, AA_5 = P\ |)$$

$$(4)$$

The chain rule, or general product rule, shown in equation (5) below permits the calculation of any member of a joint distribution of a set of random variables using only conditional probabilities. This rule may be applied to equation (4) such that the Sequence Probability can be re-expressed as shown in equation (6) below:

$$P(A \cap B) = P(A|B)P(B)$$

$$(5)$$

$$SP = P(M|\ A\ A\ E\ P). P(A|A\ E\ P). P(A|E\ P). P(E|P). P(P)$$

$$(6)$$

The first type of probability values, which are determinable using the method 100, are not the same as the conditional probability values expressed in equation (6) above. However, it is possible to modify equation (6) to determine a probability value of the second type $P_{AA\text{-}chain}$ which is an estimation of the Sequence Probability. The modified equation, shown below in equation (7), uses probability values of the first type, which are derivable using the method 100, in particular, the set of probability values 302. In the present example, where the sequence of letters is [M A A E P], the second type of probability value, $P_{AA\text{-}chain}$, is determined based on a product of the probability values, of the first type, associated with the respective letter for each position, expressed in an example below as:

$$P_{AA-chain} = P(M|AAEP). P(A|MAEP). P(A|MAEP). P(E|MAAP). P(P|MAAE)$$

$$(7)$$

The second type of probability value $P_{AA\text{-}chain}$ may be generally expressed as the equation (8) below:

$$P_{AA-chain} = \prod_{i=1}^{N} P_{i,AA_i} = \prod_{i=1}^{i=N} P(AA_i = aa_i \mid \cap_{k \neq i} AA_k = aa_k, \theta)$$

$$(8)$$

Where $AA_i$ represents a position in the amino acid chain at index i, $aa_i$ represents an amino acid found at a respective position in the amino acid chain, i.e. a wild-type amino acid, at index i, and $\theta$ represents other potential conditions imposed on the amino acid chain. Determining a product of probability values in this way may result in small probability values of the second type. Amino acid chains generally include a large number of positions, between 50 and 2000, and so calculating a product of 50 to 2000 probability values, each likely being less than one, may cause the probability values of the second type to become very small. Alternatively, the probability value of the second type may be determined based on a sum of log functions of each of the probability values associated with the respective letter in the sequence of letters for each position, as included in the set of probability values 302. In other words, the probability value of the second type may be determined by calculating a sum of log functions applied to the first type of probability values. A probability value of the second type which is calculated in this way may be referred to as a log likelihood for the amino acid chain, and is expressed in equation (9) below:

$$P_{AA-chain} = \sum_{i=1}^{N} \log(P_{i,AA_i}) = \sum_{i=1}^{N} \log(P(AA_i = aa_i \mid \cap_{k \neq i} AA_k = aa_k, \theta)$$

$$(9)$$

Determining the probability value of the second type $P_{AA-chain}$ in this way allows larger values, representing an estimation of a likelihood of the physiological plausibility of an amino acid chain to be generated. Generating larger values in this way provides information which is more easily compared between a plurality of different amino acid chains.

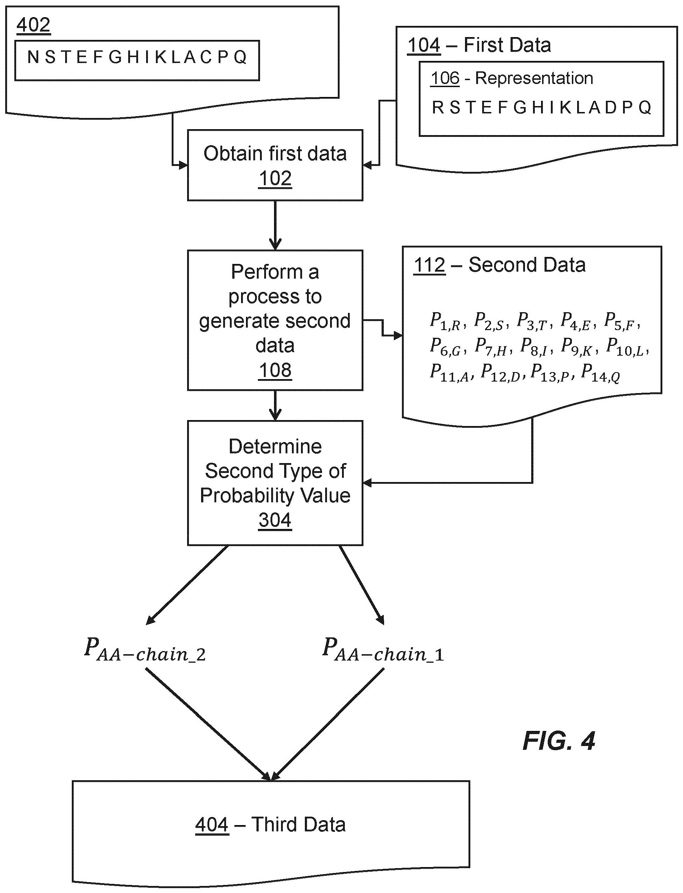

The method 100 may include generating a probability value of the second type $P_{AA-chain}$ for each of two or more amino acid chains. Figure 4 shows an example in which the probability value of the second type $P_{AA-chain}$ is a first probability value of the second type and is re-written as $P_{AA-chain1}$ and the method 100 comprises generating a second probability value of the second type $P_{AA-chain2}$ associated with an amino acid chain which is different to the amino acid chain represented by the first data 104. The amino acid chain which is different to the amino acid chain represented by the first data 104 may be represented by further data 402. In Figure 4, the method 100 comprises generating third data 404 representing a comparison of the first probability value of the second type $P_{AA-chain1}$ and the second probability value of the second type $P_{AA-chain2}$. The comparison may include an indication of which probability value represents a greater likelihood of the viability of a respective amino acid chain. Alternatively, or additionally, the data 404 may indicate which of the amino acid chains is more likely and/or an indication of a difference between the first probability value of the second type $P_{AA-chain1}$ and the second probability value of the second type $P_{AA-chain2}$. In this way, the method 100 is able to provide a comparison of the viability of two or more amino acid chains, therefore allowing researchers to quickly evaluate and identify which of two more amino acid chains are more likely, or stable, and therefore promising candidates for synthesis and/or further research.

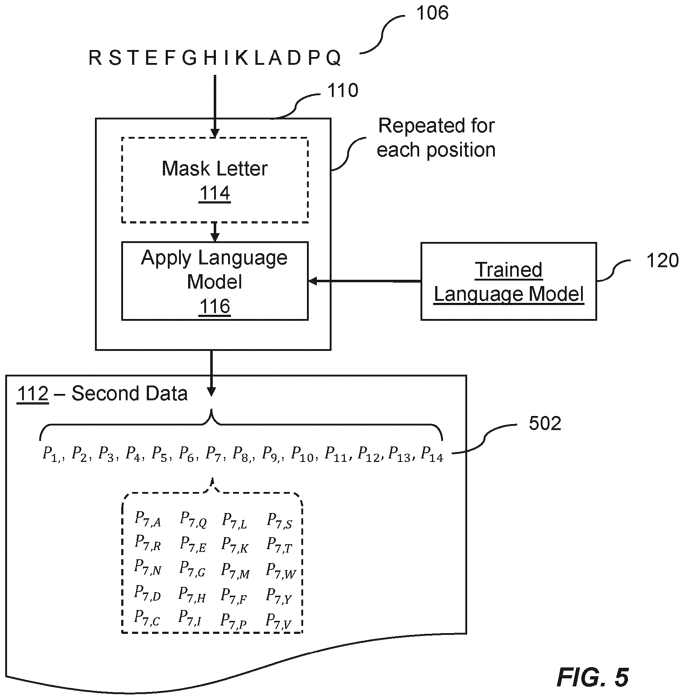

In some examples, performing the process 110 to generate the second data 112 comprises performing the process 110 for each position $AA_i$ in the sequence of letters to determine two or more probability values $P_i$, of the first type, associated with each of the positions $AA_i$, wherein each probability value associated with a position $AA_i$ is associated with a different one of the set of possible amino acids from other probability values associated with the same position $AA_i$. Figure 5 illustrates an example in which process 110 has been applied to the representation 106 to determine a set 502 of probability values comprising two or more probability values, of the first type, for each position $AA_i$ in the sequence of letters. In the example shown the set 502 of probability values comprises twenty probability values for each position $AA_i$ in the sequence of letters. Each probability value for a given position is associated with a different letter, representing a respective amino acid, of the set of possible amino acids. Generating second data 112 which includes probability values of the first type in this way provides an indication of the viability of each of the possible amino acids being present at each given location. This information provides an insight into the structure and stability of amino acid chains and allows researchers to identify amino acids in an amino acid chain, such as a protein, which may be suitable candidates for modification or further research when developing new proteins, such as in the development of drugs. For example, the first type of probability values may indicate that a potential alternative amino acid, which is not included at a particular position in the amino acid chain, may be a strong candidate for inclusion at the particular position based on a respective probability value of the first type.

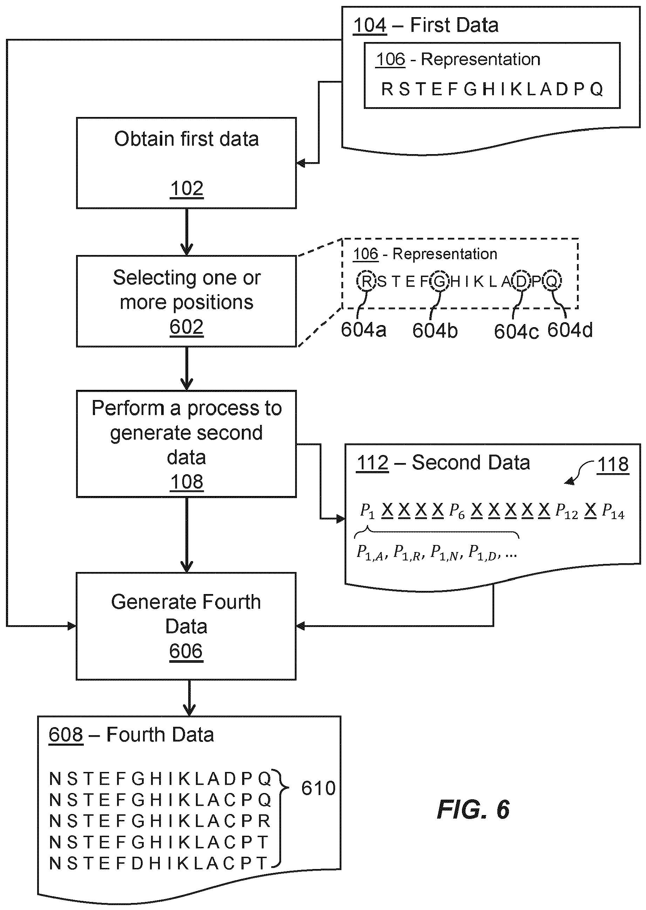

In some circumstances, the process 110 may be applied to one or more select positions in an amino acid chain. In this way, the method 100 may be targeted to specific amino acids in an amino acid chain, such as a protein, thereby saving processing power and reducing the time for evaluating the amino acid chain. Figure 6 shows an example in which the method 100 includes selecting 602 one or more positions 604a, 604b, 604c, 604d and performing the process 110 to generate the second data 112. The second data 112 being generated by performing the process 110 for the selected one or more positions 604a, 604b, 604c, 604d to determine one or more probability values for each selected position 604a, 604b, 604c, 604d. In Figure 6, the process 110 is performed for the selected one or more positions 604a, 604b, 604c, 604d to determine two or more, in this case twenty, probability values $P_1$, $P_6$, $P_{12}$, $P_{14}$ for each position 604a, 604b, 604c, 604d. Each probability value $P_{1,A}$, $P_{1,R}$, $P_{1,N}$, $P_{1,D}$ associated with a given one of the selected positions 604a is associated with a different one of the set of possible amino acids from the other probability values $P_{1,A}$, $P_{1,R}$, $P_{1,N}$, $P_{1,D}$ associated with the given position 604a.

Selecting 602 one or more positions 604a, 604b, 604c, 604d may include receiving input from a user, via a user interface, or via a suitable communication module, indicating a selection of one or more positions 604a, 604b, 604c, 604d in the representation 106 of the amino acid chain. Alternatively, the one or more positions 604a, 604b, 604c, 604d which are to be selected may be indicated in the first data 104.

The method 100 may also include, as shown in Figure 6, generating fourth data 608 comprising a representation 610 of one or more alternative amino acid chains from the first data 104 using the second data 112. The fourth data 608 shown in Figure 6 comprises a representation of five different amino acid chains which have been generated based on the first data 104 and using the second data 112. The alternative amino acid chains in the representation 610 have been generated by modifying amino acids of the amino acid chain represented 106 in the first data 104 at one or more of the selected positions 604a, 604b, 604c, 604d. The modification of the amino acids at one or more of the selected positions 604a, 604b, 604c, 604d is based on the probability values, of the first type, associated with each of the selected positions 604a, 604b, 604c, 604d, which are included in the second data 112.

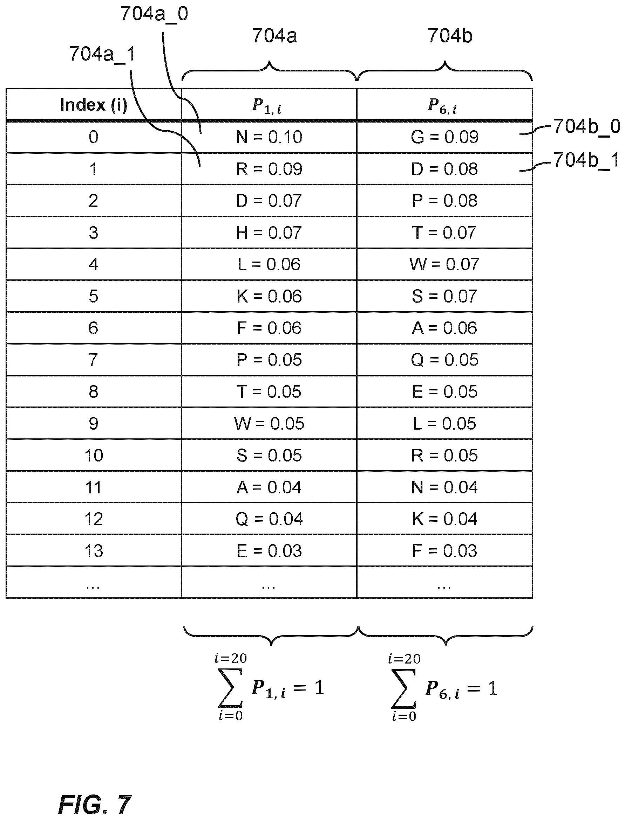

Figure 7 illustrates an example of how the fourth data 608 may be generated. In the example illustrated in Figure 7, generating the fourth data 608 comprises determining a first ordered list 704a of amino acids associated with a first selected position 604a and a second ordered list 704b of amino acids associated with a second selected position 604b. The first ordered list 704a and the second ordered list 704b are ordered according to probability values associated with each of the amino acids for the respective selected position 604a and 604b. In particular, probability values of the first type, representing a conditional probability that a given amino acid will be found at a respective position in the amino acid chain are used to order the lists of amino acids for each position. While two ordered lists 704a and 704b have been shown here, associated with two selected positions 604a and 604b, it will be appreciated that more ordered lists may be generated. Ordered lists can be generated for each selected position 604a, 604b, 604c, 604d. Alternatively, ordered lists may be generated for only a subset of the selected positions 604a, 604b, 604c, 604d. The lists 704a and 704b may have equal numbers of entries, such as where there is an entry for each possible amino acid. However, in other cases, the lists may be limited to a subset of all possible amino acids, for example, the five or ten most likely amino acids. The number of amino acids considered in each list 704a and 704b may be dependent on the variance and/or distribution of probability values associated with a respective position.

The one or more alternative amino acid chains 610 are then generated by selecting amino acids from the first ordered list 704a and the second ordered list 704b. Selecting the amino acids from the first ordered list 704a and the second ordered list 704b prioritises amino acids for each position 604a and 604b according to the associated probability values. In particular, amino acids having a higher probability value, that is a probability value indicating that the respective amino acid is more likely to occur in the amino acid chain at the respective position 604a and 604b, are prioritised. Prioritising may include starting the selection at the top of the list and selecting subsequent entries in the list when generating the set of alternative amino acid chains.

In the example shown in Figure 7 the probability values associated with a respective position 604a, 604b are normalised such that the sum of all probability values associated with the respective position 604, 604b equal one. In other examples, the probability values may not be normalised, such as where the probability values are log likelihood values.

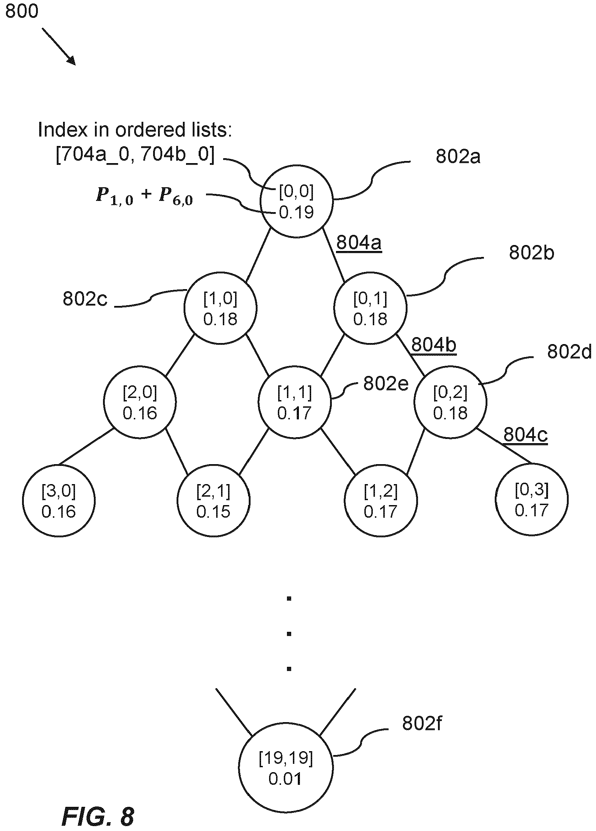

As described above in relation to Figures 6 and 7, alternative amino acid chains are generated by modifying amino acids at one or more of the selected positions 604a, 604b, 604c, and 604d in the amino acid chain represented 106 in the first data 104. In some examples, selecting amino acids from the ordered lists 704a and 704b, shown in Figure 7, which are to be used to modify amino acids at one or more of the selected positions 604a and 604b, includes generating a directed graph 800. Figure 8 shows a directed graph 800 comprising nodes 802a, 802b, 802c, 802d, 802e and edges 804a, 804b, and 804c. The directed graph 800 may alternatively be referred to as a network comprising a plurality of nodes 802a to 802e and edges 804a to 804c. Each node 802a to 802e represents a selection of an amino acid from each of the ordered lists 704a and 704b based on an index position in the ordered lists 704a and 704b. For example, a first node 802a, represents selections of amino acids from an entry at index 0 in the first list 704a and an entry at index 0 in the second list 704b. In this case, the entries, at indices [0,0], represent a selection of amino acid "N" for the first selected position 604a and a selection of amino acid "G" for the second selected position 604b. Similarly, a second node 802b represents selections of amino acids at index 0 of the first list 704a and index 1 of the second list 704b. Hence the second node 802b represents a selection of amino acid "N" for the first selected position 604a and amino acid "D" for the second selected position 604b.

An alternative amino acid chain is generated from a node 802a and 802b by modifying the wild-type amino acids at the selected positions 604a and 604b to be the selected amino acids represented by the node 802a and 802b. For example, generating an alternative amino acid from the second node 802b includes selecting the amino acid "N", to replace the wild-type amino acid "R" at the first selected position 604a, and selecting the amino acid "D" to replace the wild-type amino acid "G" at the second selected position 604b. In some cases, one of the nodes may be associated with the wild-type amino acids for each of the selection positions 604a and 604b and the alternative amino acid chain generated from this node may be excluded from the list of alternative amino acid chains.

Each node 802a to 802e is also associated with a sum of the probability values associated with the respective amino acids. The directed graph 800 represents a ranking of combinations of amino acids for the selected positions 604a and 604b according to the sum of probability values associated with the amino acids for the selected positions 604a and 604b. The first node 802a, referred to as a root node 802a, of the directed graph 800 represents a combination of amino acids for the selected positions 604a and 604b which has a maximum sum of the probability values associated with those amino acids. The end node 802f, referred to as the leaf node, or nodes, represents a combination of amino acids for the selected positions 604a and 604b which has a minimum sum of the probability values associated with those amino acids. Once the directed graph 800 has been determined, the alternative amino acid chains are generated by traversing the edges 804a, 804b, 804c and nodes 802a, 802b, 802d of the directed graph 800 and generating an alternative amino acid chain at each traversed node 802, 802b, 802d.

Traversing the directed graph includes starting at a root node 802a of the directed graph 800 and generating an amino acid chain based on the amino acids for the selected positions 604a and 604b, which are represented by the root node 802a. An edge 804a connected to the root node 802a is selected, wherein the edge 804a leads to a subsequent node 802b. Generally, a subsequent node 802b having the largest sum of associated probability values compared to an alternative subsequent node 802c, or nodes, is the selected as the subsequent node 802b. However, in some cases, two potential subsequent nodes 802b and 802c may be associated with probability values which are equal. Where the probability values associated with two potential subsequent nodes 802b and 802c are equal, one 802b of the potential subsequent nodes 802b and 802c may be selected at random. An alternative amino acid chain is then determined for the subsequent node 802b based on the amino acids for the selected positions 604a and 604b which are represented by the subsequent node 802b. Traversing the directed graph 800 in this way may be repeated from the subsequent node 802b by traversing an edge 804b leading to a further subsequent node 802d having a largest sum of associated probability values compared to an alternative subsequent node 802e. This process can be repeated a predetermined number of times to generate the set of alternative amino acid chains. For example, where twenty alternative amino acid chains are to be generated, this process may be repeated until twenty nodes have been selected.

Generating the alternative amino acid chains from the nodes 802a, 802b, and 802 d in the order in which the nodes 802a, 802b, and 802d are selected, provides a ranked list of alternative amino acid chains. These alternative amino acid chains are ranked according to the sum of the probability values associated with the amino acids represented by each node 802a, 802b, and 802d. The alternative amino acid chains which are represented in the fourth data 608 may also be ranked, thereby indicating the relative likelihoods of each of the alternative amino acid chains in the fourth data 608.

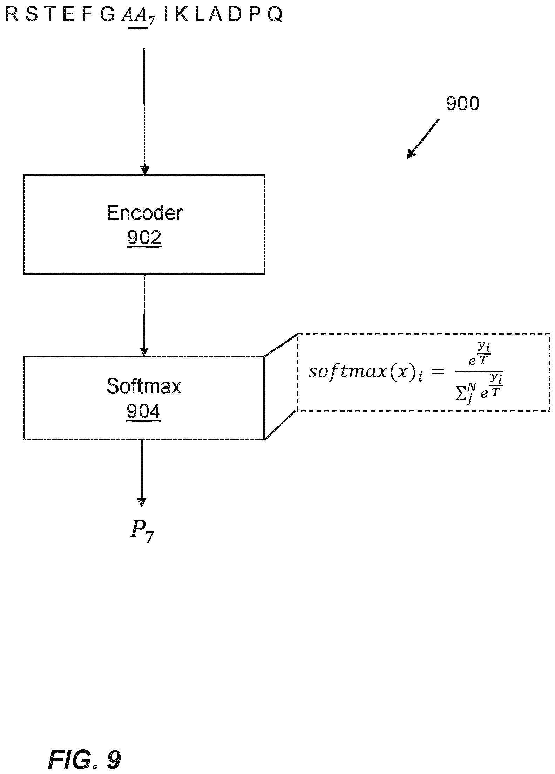

Figure 9 shows an example of the language model 120 in detail. The language model 120 comprises a Transformer model 900 including an encoder 902. The Transformer model 900 has been trained using one or more datasets representing amino acid chains. While a single encoder 902 has been shown in Figure 9, in some cases a plurality of encoders 902 may be provided. Transformer models 900 demonstrate particular utility with respect to language processing in deep learning methods. Transformer models 900 provide efficient and accurate language processing when trained appropriately and are suitable for parallelization, thereby, allowing the process to be parallelized across a plurality of processors and reducing the time needed to perform the method 100.

The Transformer model 900 may be trained based on a predictive masking task. In particular, the predictive masking task may comprise providing the Transformer model 900 with a set of masked amino acid chains, each masked amino acid chain comprising a known amino acid chain in which at least one amino acid is masked. The Transformer model 900 is then trained to identify a respective set of known amino acid chains from the set of masked amino acid chains. During the training phase, the Transformer model 900 may include further components, such as a decoder. The known amino acid chains are derived from datasets, such as UniRef100, comprising representations of amino acid chains, such as proteins.

The output of the Transformer 900 is provided to a softmax function 904. Softmax functions are generally functions which take a vector of real numbers and normalise the vector to a probability distribution. The softmax function 904 in Figure 9 is dependent on a temperature value T. An example of a softmax function is expressed below in equation (10):

$$softmax(x)_i = \frac{e^{\frac{y_i}{T}}}{\sum_j^N e^{\frac{y_i}{T}}}$$

$$(10)$$

By making the softmax function dependent on a temperature it is possible to alter the relative distribution of probability values $P_{i,AA}$ output from the language model 120. For example, when calculating a plurality of probability values $P_7$ comprising a probability value associated with each of the set of possible amino acids it is possible that one amino acid may dominate the probability distribution and hence make it more difficult to determine information from the remaining probability values. In this case a high temperature value may be used in the softmax function to increase the noise and thereby reduce the dominance of one particular probability value. Alternatively, in examples where it is difficult to identify one amino acid which dominates with respect to probability, the temperature value may be set to a low value to decrease the noise and thereby allow more information to be determined from the probability values and to identify one or more prominent amino acids. In some examples, the method 100 includes obtaining a selection of a temperature value for use in the softmax function 904. The selection of a temperature value may be a static selection or may be updated based on an evaluation of a variance between the probability values associated with a given position in the sequence of letters.

*Implementation Details*

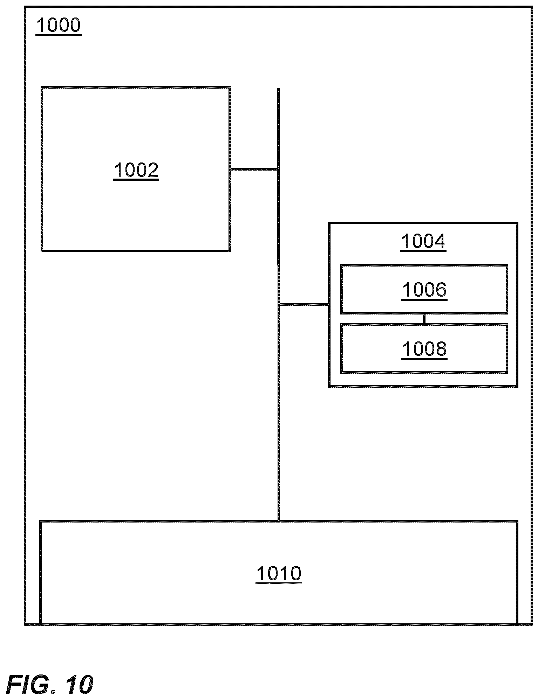

Figure 10 shows an example of a computer system according to the present disclosure. The computer system comprises at least one processor 1002 and at least one storage 1004. The at least one processor 1002 may include any suitable combination of processing units such as Central Processing Units, Graphics Processing Units, Neural Processing Units, and any other suitable general purpose or system specific processing units. The storage 1004 may include any suitable combination of volatile and nonvolatile memory. The storage 1004 includes a trained language model 1006 which has been trained using one or more datasets representing amino acid chains and computer-executable instructions 1008. The computer-executable instructions 1008, when executed by the at least one processor 1002 cause the at least one processor 1002 to implement a method 100 according to the examples described above. The computer system 1000 may additionally include a user interface 1010 for displaying information to a user and receiving input from the user. The computer system 1000 may be a distributed computing system comprising a plurality of individual computing units which are communicatively coupled, either by wireless, or wired means. For example, individual computing units may be coupled via a local area network, LAN, and/or a wide area network, WAN.

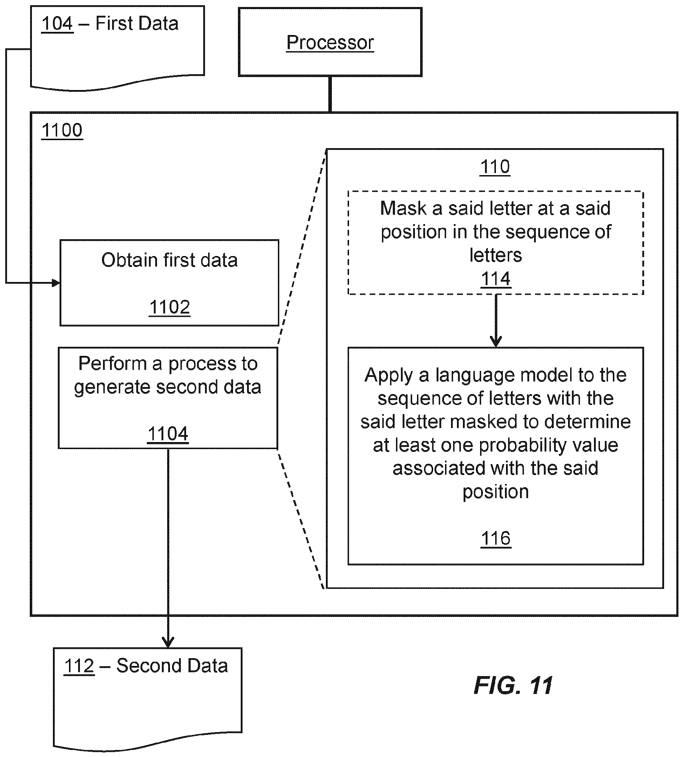

Figure 11 shows a non-transitory computer-readable storage medium 1100 comprising computer-executable instructions 1102 and 1104 which, when executed by one or more processors, cause the processors to perform a method 100 according to the examples described above with respect to Figures 1 to 9.

The above embodiments are to be understood as illustrative examples of the invention. Further embodiments of the invention are envisaged. For example, the language model 120 may be an artificial recurrent neural network such as a long short-term memory, LSTM, model. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

*Numbered Clauses*

The following numbered clauses describe various embodiments of the present disclosure:

1. A computer-implemented method for evaluating an amino acid chain, the computer implemented method comprising:

obtaining first data, wherein the first data includes a representation of an amino acid chain, the representation comprising a sequence of two or more letters, wherein each letter of the sequence of letters corresponds to a respective amino acid of a set of possible amino acids and a position of each letter in the sequence of letters represents a respective position of a said amino acid in the amino acid chain; and

performing a process to generate second data, the second data comprising a set of one or more probability values associated with at least one position in the amino acid chain, the process comprising, for a said position in the sequence of letters, applying a language model to the sequence of letters to determine at least one probability value associated with the said position,

wherein the language model is trained using one or more datasets representing amino acid chains.

2. The computer-implemented method of clause 1, wherein performing the process to generate second data comprises performing the process for each position in the sequence of letters to determine one or more probability values for each position.

3. The computer-implemented method of clause 1, wherein performing the process to generate second data comprises performing the process for each position in the sequence of letters to determine two or more probability values for each position, wherein each probability value associated with a said position is associated with a different one of the set of possible amino acids from other probability values associated with the said position.

4. The computer-implemented method of clause 1, wherein performing the process to generate second data comprises performing the process for each position in the sequence of letters to determine one or more probability values for each position, wherein the one or more probability value for each position are probability values of a first type and include probability values associated with a respective letter in the sequence of letters for each position, and wherein the method comprises determining a probability value of a second type based on the probability values associated with the respective letter for each position.

5. The computer-implemented method of clause 4, wherein the probability value of the second type is determined based on a product of the probability values associated with the respective letter for each position.

6. The computer-implemented method of clause 4, wherein the probability value of the second type is determined based on a sum of log functions of each of the probability values associated with the respective letter for each position.

7. The computer-implemented method of any of clauses 4 to 6, wherein the probability value of the second type is a first probability value of the second type, and the method further comprises:

generating a second probability value of the second type associated with an amino acid chain which is different to the amino acid chain represented in the first data; and

generating third data representing a comparison of the first probability value of the second type and the second probability value of the second type.

8. The computer-implemented method of clause 1, wherein the method comprises selecting one or more positions, and wherein the step of performing the process to generate second data comprises performing the process for the selected one or more positions to determine one or more probability value for each selected position.

9. The computer-implemented method of clause 8, wherein performing the process to generate the second data comprises performing the process for the selected one or more positions to determine two or more probability values for each position, wherein each probability value associated with a said position is associated with a different one of the set of possible amino acids from the other probability values associated with the said position.

10. The computer-implemented method of clause 9, wherein the method further comprises generating fourth data comprising a representation of one or more alternative amino acid chains from the first data using the second data.

11. The computer-implemented method of clause 10, wherein generating the fourth data comprises determining one or more alternative amino acid chains by:

determining a first ordered list of amino acids associated with a first selected position, the first ordered list being ordered according to probability values associated with each of the amino acids for the first selected position;

determining a second ordered list of amino acids associated with a second selected position, the second ordered list being ordered according to probability values associated with each of the amino acids for the selected position; and

generating one or more alternative amino acid chains by selecting amino acids from the first ordered list and the second ordered list, wherein the selection prioritises amino acids for each position according to the associated probability values.

12. The computer-implemented method of any preceding clause, wherein performing the process for the said position comprises masking a said letter at the said position and wherein applying the language model to the sequence of letters includes applying the language model to the sequence of letters with the said letter masked.

13. The computer-implemented method of any preceding clause, wherein the applying the language model comprises selecting the language model from a set of one or more language models.

14. The computer-implemented method of clause 13, wherein the language model is selected based on the first data.

15. The computer-implemented method of any preceding clause, wherein the language model comprises a Transformer model including at least an encoder and trained using the one or more datasets representing amino acid chains.

16. The computer-implemented method of clause 15, wherein the Transformer model is trained by:

   providing the Transformer model with a set of masked amino acid chains, each masked amino acid chain comprising a known amino acid chain in which at least one amino acid is masked; and

   training the Transformer model to identify a respective set of known amino acid chains.

17. The computer-implemented method of clause 15 or clause 16, wherein an output of the Transformer model is input to a softmax function, and wherein the softmax function is dependent on a temperature value.

18. The computer-implemented method of clause 17, wherein the method comprises obtaining a selection of a temperature value for use in the softmax function.

19. A computer system comprising at least one processor and at least one storage, the storage including:

   a trained language model which has been trained using one or more datasets representing amino acid chains; and

   computer-executable instructions which, when executed by the at least one processor, cause the computer system to perform a computer-implemented method according to any one of clauses 1 to 18.

20. The computer system of clause 19, wherein the computer system includes one or more user interfaces.

21. A non-transitory computer-readable storage medium comprising computer-executable instructions which, when executed by one or more processors, cause the processors to perform a computer-implemented method according to any one of clauses 1 to 18.

## Claims

1. A computer-implemented method for evaluating an amino acid chain, the computer implemented method comprising:

   obtaining first data, wherein the first data includes a representation of an amino acid chain, the representation comprising a sequence of two or more letters, wherein each letter of the sequence of letters corresponds to a respective amino acid of a set of possible amino acids and a position of each letter in the sequence of letters represents a respective position of a said amino acid in the amino acid chain; and
   performing a process to generate second data, the second data comprising a set of one or more probability values associated with at least one position in the amino acid chain, the process comprising, for a said position in the sequence of letters, applying a language model to the sequence of letters to determine at least one probability value associated with the said position,
   wherein the language model is trained using one or more datasets representing amino acid chains.

2. The computer-implemented method of claim 1, wherein performing the process to generate second data comprises performing the process for each position in the sequence of letters to determine one or more probability values for each position.

3. The computer-implemented method of claim 1, wherein performing the process to generate second data comprises performing the process for each position in the sequence of letters to determine two or more probability values for each position, wherein each probability value associated with a said position is associated with a different one of the set of possible amino acids from other probability values associated with the said position.

4. The computer-implemented method of claim 1, wherein performing the process to generate second data comprises performing the process for each position in the sequence of letters to determine one or more probability values for each position, wherein the one or more probability value for each position are probability values of a first type and include probability values associated with a respective letter in the sequence of letters for each position, and wherein the method comprises determining a probability value of a second type based on the probability values associated with the respective letter for each position; and
optionally, wherein the probability value of the second type is determined based on:

a product of the probability values associated with the respective letter for each position; or
a sum of log functions of each of the probability values associated with the respective letter for each position.

5. The computer-implemented method of claim 4, wherein the probability value of the second type is a first probability value of the second type, and the method further comprises:

generating a second probability value of the second type associated with an amino acid chain which is different to the amino acid chain represented in the first data; and
generating third data representing a comparison of the first probability value of the second type and the second probability value of the second type.

6. The computer-implemented method of claim 1, wherein the method comprises selecting one or more positions, and wherein the step of performing the process to generate second data comprises performing the process for the selected one or more positions to determine one or more probability value for each selected position.

7. The computer-implemented method of claim 6, wherein performing the process to generate the second data comprises performing the process for the selected one or more positions to determine two or more probability values for each position, wherein each probability value associated with a said position is associated with a different one of the set of possible amino acids from the other probability values associated with the said position; and
optionally, wherein the method further comprises generating fourth data comprising a representation of one or more alternative amino acid chains from the first data using the second data.

8. The computer-implemented method of claim 7, wherein generating the fourth data comprises determining one or more alternative amino acid chains by:

determining a first ordered list of amino acids associated with a first selected position, the first ordered list being ordered according to probability values associated with each of the amino acids for the first selected position;
determining a second ordered list of amino acids associated with a second selected position, the second ordered list being ordered according to probability values associated with each of the amino acids for the selected position; and
generating one or more alternative amino acid chains by selecting amino acids from the first ordered list and the second ordered list, wherein the selection prioritises amino acids for each position according to the associated probability values.

9. The computer-implemented method of any preceding claim, wherein performing the process for the said position comprises masking a said letter at the said position and wherein applying the language model to the sequence of letters includes applying the language model to the sequence of letters with the said letter masked.

10. The computer-implemented method of any preceding claim, wherein the applying the language model comprises selecting the language model from a set of one or more language models; and
optionally, wherein the language model is selected based on the first data.

11. The computer-implemented method of any preceding claim, wherein the language model comprises a Transformer model including at least an encoder and trained using the one or more datasets representing amino acid chains; and
optionally, wherein an output of the Transformer model is input to a softmax function, and wherein the softmax function is dependent on a temperature value.

12. The computer-implemented method of claim 11, wherein the Transformer model is trained by:

providing the Transformer model with a set of masked amino acid chains, each masked amino acid chain comprising a known amino acid chain in which at least one amino acid is masked; and

training the Transformer model to identify a respective set of known amino acid chains.

13. The computer-implemented method of claim 11 or claim 12, wherein the method comprises obtaining a selection of a temperature value for use in the softmax function.

14. A computer system comprising at least one processor and at least one storage, the storage including:

a trained language model which has been trained using one or more datasets representing amino acid chains; and computer-executable instructions which, when executed by the at least one processor, cause the computer system to perform a computer-implemented method according to any one of claims 1 to 13, wherein, optionally, the computer system includes one or more user interfaces.

15. A non-transitory computer-readable storage medium comprising computer-executable instructions which, when executed by one or more processors, cause the processors to perform a computer-implemented method according to any one of claims 1 to 13.

100

104 – First Data

106 - Representation

R S T E F G H I K L A D P Q

Obtain first data

102

Perform a process to generate second data

108

110

Mask a said letter at a said position in the sequence of letters

114

Apply a language model to the sequence of letters to determine at least one probability value associated with the said position

116

112 – Second Data

118

$$P_1 \ \underline{X} \ \underline{X} \ \underline{X} \ \underline{X} \ P_6 \ \underline{X} \ \underline{X} \ \underline{X} \ \underline{X} \ \underline{X} \ P_{12} \ \underline{X} \ P_{14}$$

$$P_{1,A}, P_{1,R}, P_{1,N}, P_{1,D}, \dots$$

*FIG. 1*

_110_

_106_

R S T E F G H I K L A D P Q

R S T E F G _AA_$_7$ I K L A D P Q

| Trained Language Model | _120_ |

$P_7$

$$\begin{Bmatrix} P_{7,A} & P_{7,Q} & P_{7,L} & P_{7,S} \\ P_{7,R} & P_{7,E} & P_{7,K} & P_{7,T} \\ P_{7,N} & P_{7,G} & P_{7,M} & P_{7,W} \\ P_{7,D} & P_{7,H} & P_{7,F} & P_{7,Y} \\ P_{7,C} & P_{7,I} & P_{7,P} & P_{7,V} \end{Bmatrix}$$

*FIG. 2*

R S T E F G H I K L A D P Q   $\overset{\displaystyle\frown}{\phantom{x}}$ 106

Repeated for each position

**Mask Letter**
**114**

**Apply Language Model**
**116**

110

**Trained Language Model** — 120

$P_{1,R},\ P_{2,S},\ P_{3,T},\ P_{4,E},\ P_{5,F},\ P_{6,G},\ P_{7,H},\ P_{8,I},\ P_{9,K},\ P_{10,L},\ P_{11,A},\ P_{12,D},\ P_{13,P},\ P_{14,Q}$ $\Big\}$ 302

**Determine a Second Type of Probability Value**
**304**

$P_{AA-chain}$

*FIG. 3*

**402**

N S T E F G H I K L A C P Q

**104** – First Data

**106** - Representation

R S T E F G H I K L A D P Q

Obtain first data
**102**

Perform a process to generate second data
**108**

**112** – Second Data

$P_{1,R}$, $P_{2,S}$, $P_{3,T}$, $P_{4,E}$, $P_{5,F}$, $P_{6,G}$, $P_{7,H}$, $P_{8,I}$, $P_{9,K}$, $P_{10,L}$, $P_{11,A}$, $P_{12,D}$, $P_{13,P}$, $P_{14,Q}$

Determine Second Type of Probability Value
**304**

$P_{AA-chain\_2}$

$P_{AA-chain\_1}$

*FIG. 4*

**404** – Third Data

106

R S T E F G H I K L A D P Q

110

```
┌─────────────────────────────┐
│ ┌ ─ ─ ─ ─ ─ ─ ─ ─ ─ ─ ─ ┐   │     Repeated for
│ │      Mask Letter      │   │     each position
│ │        114            │   │
│ └ ─ ─ ─ ─ ─ ─ ─ ─ ─ ─ ─ ┘   │
│ ┌───────────────────────┐   │
│ │    Apply Language      │   │  ◄── ┌──────────────────┐      120
│ │       Model            │   │      │     Trained      │
│ │        116             │   │      │ Language Model   │
│ └───────────────────────┘   │      └──────────────────┘
└─────────────────────────────┘
```

112 – Second Data

502

$$P_1, P_2, P_3, P_4, P_5, P_6, P_7, P_8, P_9, P_{10}, P_{11}, P_{12}, P_{13}, P_{14}$$

$$
\begin{array}{cccc}
P_{7,A} & P_{7,Q} & P_{7,L} & P_{7,S} \\
P_{7,R} & P_{7,E} & P_{7,K} & P_{7,T} \\
P_{7,N} & P_{7,G} & P_{7,M} & P_{7,W} \\
P_{7,D} & P_{7,H} & P_{7,F} & P_{7,Y} \\
P_{7,C} & P_{7,I} & P_{7,P} & P_{7,V}
\end{array}
$$

*FIG. 5*

104 – First Data

106 - Representation

R S T E F G H I K L A D P Q

Obtain first data

102

Selecting one or
more positions
602

106 - Representation

®S T E F G̈H I K L A ©D̈ ©P̈ ©Q̈

604a    604b       604c  604d

Perform a process to
generate second
data
108

112 – Second Data    118

$P_1$ X X X X $P_6$ X X X X X $P_{12}$ X $P_{14}$

$P_{1,A}, P_{1,R}, P_{1,N}, P_{1,D}, ...$

Generate Fourth
Data
606

608 – Fourth Data

N S T E F G H I K L A D P Q
N S T E F G H I K L A C P Q
N S T E F G H I K L A C P R    610
N S T E F G H I K L A C P T
N S T E F D H I K L A C P T

*FIG. 6*

| Index (i) | $P_{1,i}$ | $P_{6,i}$ |
|-----------|-----------|-----------|
| 0 | N = 0.10 | G = 0.09 |
| 1 | R = 0.09 | D = 0.08 |
| 2 | D = 0.07 | P = 0.08 |
| 3 | H = 0.07 | T = 0.07 |
| 4 | L = 0.06 | W = 0.07 |
| 5 | K = 0.06 | S = 0.07 |
| 6 | F = 0.06 | A = 0.06 |
| 7 | P = 0.05 | Q = 0.05 |
| 8 | T = 0.05 | E = 0.05 |
| 9 | W = 0.05 | L = 0.05 |
| 10 | S = 0.05 | R = 0.05 |
| 11 | A = 0.04 | N = 0.04 |
| 12 | Q = 0.04 | K = 0.04 |
| 13 | E = 0.03 | F = 0.03 |
| … | … | … |

704a_0  704a_1  704a  704b  704b_0  704b_1

$$\sum_{i=0}^{i=20} P_{1,i} = 1 \qquad \sum_{i=0}^{i=20} P_{6,i} = 1$$

*FIG. 7*

800

Index in ordered lists:
[704a_0, 704b_0]

$P_{1,0} + P_{6,0}$

802a

[0,0]
0.19

804a

802b

[0,1]
0.18

802c

[1,0]
0.18

804b

802d

[0,2]
0.18

[2,0]
0.16

[1,1]
0.17

802e

804c

[3,0]
0.16

[2,1]
0.15

[1,2]
0.17

[0,3]
0.17

.
.
.

802f

[19,19]
0.01

*FIG. 8*

R S T E F G $AA_7$ I K L A D P Q

900

Encoder
902

Softmax
904

$$softmax(x)_i = \frac{e^{\frac{y_i}{T}}}{\sum_j^N e^{\frac{y_i}{T}}}$$

$P_7$

FIG. 9

**FIG. 10**

104 – First Data

Processor

1100

Obtain first data

1102

Perform a process to generate second data

1104

110

Mask a said letter at a said position in the sequence of letters

114

Apply a language model to the sequence of letters with the said letter masked to determine at least one probability value associated with the said position

116

112 – Second Data

*FIG. 11*

## EUROPEAN SEARCH REPORT

Europäisches Patentamt
European Patent Office
Office européen des brevets

**Application Number**

EP 22 17 5892

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | INGRAHAM JOHN ET AL: "Generative models for graph-based protein design", 33RD CONFERENCE ON NEURAL INFORMATION PROCESSING SYSTEMS (NEURIPS 2019), 27 March 2019 (2019-03-27), pages 1-12, XP055832397, | 1-8,10, 11,13-15 | INV. G16B30/00 G16B40/20 |
| A | * Abstract, Chapter 1 Introduction (Par. 2), Section 2.2 Structured Transformer (Par. 1, subsection "Encoder"), Section 4.1 Statistical comparison to likelihood-based models (Par. 3), Section 4.2 Benchmarking protein redesign (Par. 1), Section 4.3 Unsupervised anomaly detection for experimental protein design (Par 1); figure 1 * | 9,12 | |
| X | ALI MADANI ET AL: "ProGen: Language Modeling for Protein Generation", ARXIV.ORG, CORNELL UNIVERSITY LIBRARY, 201 OLIN LIBRARY CORNELL UNIVERSITY ITHACA, NY 14853, 8 March 2020 (2020-03-08), XP081638520, | 1-8,10, 11,13-15 | |
| A | * Abstract, 1. Introduction (Par. 4), 3. Methods (Par. 1; Page 3, column 1), * | 9,12 | **TECHNICAL FIELDS SEARCHED (IPC)** G16B |

-/--

The present search report has been drawn up for all claims

| Place of search | Date of completion of the search | Examiner |
|---|---|---|
| Munich | 30 September 2022 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Europäisches Patentamt
European Patent Office
Office européen des brevets

Application Number

EP 22 17 5892

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RIVES ALEXANDER ET AL: "Biological structure and function emerge from scaling unsupervised learning to 250 million protein sequences", PROCEEDINGS OF THE NATIONAL ACADEMY OF SCIENCES, [Online] vol. 118, no. 15, 5 April 2021 (2021-04-05), XP55966249, ISSN: 0027-8424, DOI: 10.1073/pnas.2016239118 [retrieved on 2022-09-29] | 1-12,14, 15 | |
| A | * Abstract, Introduction (last paragraph), Background (last paragraph), Scaling Language Models to 250 Million Diverse Protein Sequences (entire section), Multiscale Organization in Sequence Representations (Learning Encodes Biochemical Properties). * | 13 | |
| A,P | OSADCHY MARGARITA ET AL: "How Deep Learning Tools Can Help Protein Engineers Find Good Sequences", JOURNAL OF PHYSICAL CHEMISTRY PART B, [Online] vol. 125, no. 24, 9 June 2021 (2021-06-09) , pages 6440-6450, XP55966602, US ISSN: 1520-6106, DOI: 10.1021/acs.jpcb.1c02449 Retrieved from the Internet: URL:https://pubs.acs.org/doi/pdf/10.1021/a cs.jpcb.1c02449> [retrieved on 2022-09-29] * Section "Autoregressive models"; page 3 - page 4 * | 1-15 | TECHNICAL FIELDS SEARCHED (IPC) |

The present search report has been drawn up for all claims

| Place of search | Date of completion of the search | Examiner |
|---|---|---|
| Munich | 30 September 2022 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)